# EUROPEAN PATENT APPLICATION

(11) **EP 2 409 678 A1**
(43) Date of publication of application: **25.01.2012**
(21) Application number: 10753570.0
(22) Date of filing: 18.03.2010
(51) Int. Cl.: A61K 8/31, A45D 44/00, A61K 8/02, A61K 8/89, A61Q 1/02

(54) **SOLVENT COMPOSITION FOR CONVERSION TO MAKEUP COSMETIC**

(30) Priority: 19.03.2009 JP 2009069088
(71) Applicant: Shiseido Co., Ltd., Tokyo 104-8010 (JP)
(72) Inventor: YAMAGUCHI, Kanako, Yokohama-shi Kanagawa 224-8558 (JP); YOKOO, Mihoshi, Yokohama-shi Kanagawa 236-8643 (JP)
(74) Representative: Gendron, Vincent Christian
(86) International application number: PCT/JP2010/054643
(87) International publication number: WO 2010/107079

(57) **Abstract**

Problem: To provide a technique of simply and easily convert a powder makeup cosmetic such as powdery foundation or the like into a pickering-emulsificated liquid makeup cosmetic such as liquid foundation or the like.

Means for Resolution: A solvent composition for use as a dispersion medium for converting a powder makeup cosmetic into a pickering-emulsificated liquid makeup cosmetic, which comprises (a) from 0.1 to 65% by mass of an aqueous component, and (b) from 35 to 99.9% by mass of one or more oily components selected from hydrocarbon oils and silicone oils.

## Description

### TECHNICAL FIELD

The present invention relates to a solvent composition to be used for dispersing a powder makeup cosmetic in the solvent composition to thereby convert the powder makeup cosmetic into a pickering-emulsificated liquid makeup cosmetic having a superior feeling in use.

### BACKGROUND ART

A powder makeup cosmetic, typified by a powder-solid-state powdery foundation, has functions of skin color correction, texture modification, covering spots and freckles, and protection from extrinsic stimuli such as ultraviolet(=UV) rays, etc. The powder makeup cosmetic includes various others than the above-mentioned powdery foundations, for example, powdery base makeup cosmetics such as face powder (e.g., pressed powder, loose powder), and powdery point makeup cosmetics (e.g., cheek rouge, eye shadow, etc.).

From the point of product characteristics that one of the purposes of the cosmetic foundation lies in skin color correction, it may be sometimes desired to change the tone of color of the purchased powdery foundation. In such a case, discarding the powdery foundation or leftovers thereof that will be no more used or will be unnecessary any more would result in a waste of resources and is unfavorable from the environmental viewpoint.

Also, there is another demand for changing the base and the form of makeup cosmetics in accordance with the season. For example, there may be the case where a powdery foundation is ordinarily used, but only in the winter season in which the skin tends to be dried, a pickering-emulsificated type liquid foundation (e.g., liquid foundation, etc.) is desired to use. In such a case, if one can easily convert the purchased powdery foundation into a liquid foundation in the winter season, then it may be advantageous in that the powdery foundation singly can satisfy the above-mentioned demand, even though both a powder foundation and a liquid foundation are not purchased.

Further, among powder makeup cosmetics, specifically solidified powder cosmetics, such as powdery foundation, pressed powder or the like, are generally press-molded and contained in compact cases. Those are excellent in portability but on the other hand, when the compacts are dropped down or are hit against some other matters, the contents thereof, i.e., the solidified powder foundation, may be broken. In addition, when the remaining amount of the contents becomes small, then the remaining solidified powder foundation may remain in the peripheral edge of the compact case, and it is difficult to completely use up these leftovers, thereby a problem may occur of a waste of the resources.

Accordingly, when such an unnecessary or unused, solidified powder foundation could be converted into a liquid-type one easily and with economy, then it would reduce the waste of resources and would be favorable from the viewpoint of the global environment.

Heretofore, there is known an attempt at home to mix and knead a broken, solidified powder foundation with a lotion on hand added thereto, thereby to use it as a liquid-like foundation; however, it is apt to be uneven on applying to the skin therewith, and therefore could not provide a sufficient effect both in point of the feeling in use and the covering ability thereof, and it actually could not fully exhibit the function thereof.

A technique of easily converting a purchased powdery foundation into a liquid foundation only in the winter season, and a solvent composition for use for converting an unnecessary or unused powdery foundation into a liquid foundation have heretofore been not created in the cosmetics industry; and the present invention is a quite novel and creative technique. Accordingly, there exists no specific prior-art reference information to be described here, relating to the present invention.

### SUMMARY OF THE INVENTION

### PROBLEMS THAT THE INVENTION IS TO SOLVE

The present invention has been made for the purpose of solving the problems mentioned above, and its object is to provide a technique of simply and easily converting a powder makeup cosmetic such as powdery foundation or the like into a pickering-emulsificated liquid makeup cosmetic such as liquid foundation or the like.

### MEANS FOR SOLVING THE PROBLEMS

For solving the above-mentioned problems, the present invention provides a solvent composition for use as a dispersion medium for converting a powder makeup cosmetic into a pickering-emulsificated liquid cosmetic, which comprises (a) from 0.1 to 65% by mass of an aqueous component, and (b) from 35 to 99.9% by mass of one or more oily components selected from hydrocarbon oils and silicone oils.

The invention also provides the solvent composition, which further comprises (c) a film forming agent.

The invention also provides the solvent composition, which further comprises (d) a dispersant.

The invention also provides a method for conversion of a powder makeup cosmetic, which comprises mixing the above-mentioned solvent composition and a powder makeup cosmetic to thereby convert the powder makeup cosmetic into a pickering-emulsificated liquid makeup cosmetic.

The invention also provides a cosmetic conversion kit for converting a powder makeup cosmetic into a pickering-emulsificated liquid makeup cosmetic, which comprises a container containing therein the above-mentioned solvent composition, and an another container for a temporarily storage of the powder makeup cosmetic therein.

The invention also provides a cosmetic conversion kit for converting a powder makeup cosmetic into a pickering-emusificated liquid makeup cosmetic, which comprises a container containing therein the above-mentioned solvent composition; an another container containing therein a powder makeup cosmetic; a mixing container for mixing a part or all of the powder makeup cosmetic in the container and the above solvent composition; and a spatula-like mixing member.

### ADVANTAGE OF THE INVENTION

According to the invention, a powder makeup cosmetic such as powdery foundation can be simply and easily converted into a pickering-emulsificated liquid makeup cosmetic such as liquid foundation excellent in feeling in use.

### BRIEF DESCRIPTION OF THE DRAWINGS

[Fig. 1] It is a view showing a cosmetic conversion kit of a first embodiment for use in the present invention, and Fig. 1(A) and (B) are kit constituting parts.
[Fig. 2] It is an explanatory view showing one use embodiment (use process) of the cosmetic conversion kit of the first embodiment for use in the present invention.
[Fig. 3] It is an explanatory view showing another use embodiment (use process) of the cosmetic conversion kit of the first embodiment for use in the present invention.
[Fig. 4] It is a view showing a cosmetic conversion kit of a second embodiment for use in the present, and Fig. 1(A) to (D) are kit constituting parts.

### MODE FOR CARRYING OUT THE INVENTION

The present invention is a technique of dispersing a powder makeup cosmetic, such as powdery foundation or the like, in a solvent composition to thereby convert it into a pickering-emulsificated liquid makeup cosmetic, such as liquid foundation or the like.

### [Powder Makeup Cosmetic]

In the present invention, the powder makeup cosmetic to be prepared so as to be converted into a pickering-emulsificated liquid makeup cosmetic include any and every powder makeup cosmetic commercially available in this technical field, and is not specifically defined. In concrete terms, there may be exemplified by powdery base makeup cosmetics, such as powdery foundation, face powder (e.g., pressed powder, loose powder); and powdery point makeup cosmetics, such as cheek rouge, eye shadow; but not limited thereto.

In the present invention, it is preferably used a purchased, commercial powdery foundation that has been broken while in use, or of which the remaining parts thereof have become small while in use and have remained in the inner peripheral area in the compact case. It may also be favorably used an unused powder makeup cosmetic or a one which is in use but will be no more used for the reason that the one having different color is desired. Further as described below, a cosmetic conversion kit (see "cosmetic conversion kit of a second embodiment" below) comprising a combination of a powder makeup cosmetic (especially loose powder or the like), a solvent composition, a saucer (mixing container) and a spatula (spatula-like mixing member) may be sold on the market, and the powder makeup cosmetic of the constitutive part of this kit may be used.

The powder makeup cosmetic may be one that is almost powder by itself, or one prepared by adding a binder (oil, surfactant, etc.) to a powder followed by shaping it into solidified powder cosmetic. The powder includes body pigments, such as mica, talc, kaolin, calcium carbonate, magnesium carbonate, silicic anhydride, aluminium oxide, and barium sulfate; white pigments, such as titanium dioxide, and zinc oxide; color pigments, such as red iron oxide, yellow iron oxide, black iron oxide, chromium oxide, ultramarine, prussian blue, and carbon black; pearlescent pigments, such as titanium mica, scaly flakes, and bismuth oxychloride. The binder includes oils, such as squalane, synthetic ester oils, and higher fatty acids; surfactants, such as sorbitan fatty acid esters. However, these exemplifications are not limited thereto.

The shaped and solid powder makeup cosmetic, such as powdery foundation, pressed powder or the like, comprises a powder and a binder and is contained in a compact case. The powder makeup cosmetic may be in any form of a solidified powder cosmetic, a powdery solid emulsified cosmetic or the like, for which the blend ratio of the powder ingredient and the oil ingredient may be changed or water or an emulsifier may be added; and in the present invention, the powder makeup cosmetic having any of those forms may also be used.

Hereinafter, the present invention is subscribed below in which by using a powdery foundation is described as one typical example of powder makeup cosmetic.

### [Solvent Composition]

The solvent composition of the present invention is used as a dispersion medium for converting the above-mentioned powdery foundation into a liquid foundation, and comprises (a) from 0.1 to 65% by mass of an aqueous component and (b) from 35 to 99.9% by mass of one or more oily components selected from hydrocarbon oils and silicone oils.

As component (a), herein usable is any and every other aqueous component usable in cosmetics than water. The component includes moisturizers, water-soluble polymers, drugs (whitening agents, etc.), and UV absorbents, but not limited thereto.

Examples of the moisturizer include polyethylene glycol, propylene glycol, glycerin, 1,3-butylene glycol, xylitol, sorbitol, maltitol, chondroitin sulfate, hyaluronic acid, mucoitin sulfate, caronic acid, atelocollagen, trehalose, cholesteryl 12-hydroxystearate, sodium lactate, bile salts, dl-pyrrolidonecarboxylates, short-chain soluble collagen, diglycerin (EO)PO adducts, Rosa roxburghii extract, Achillea millefolium extract, and melilot extract.

Examples of the water-soluble polymer include natural water-soluble polymers (e.g., carrageenan, xanthan gum, dextran, collagen, casein, and gelatin); semi-synthetic water-soluble polymers (e.g., starch-type water-soluble polymers such as carboxymethyl starch; cellulose-type water-soluble polymers such as methyl cellulose, hydroxyethyl cellulose, hydroxypropyl cellulose, and carboxymethyl cellulose (CMC); alginate-type water-soluble polymers such as sodium alginate); synthetic water-soluble polymers (e.g., vinyl-type water-soluble polymers, such as polyvinyl alcohol, polyvinyl pyrrolidone, and carboxyvinyl polymer; polyoxyethylene-type water-soluble polymers such as polyethylene glycol; copolymer-type water-soluble polymers such as polyoxyethylene/polyoxypropylene copolymer; acryl-type water-soluble polymers such as sodium polyacrylate, polyacrylamide); and inorganic water-soluble polymers (e.g., bentonite, laponite, and hectorite).

Examples of the drug include whitening agents (e.g., glutathion, Saxifraga stolonifera extract, arbutin, ascorbic acid and its derivatives), blood circulation promoters (e.g., benzyl nicotinate, capsaicin, tannic acid, α-borneol, tocopherol nicotinate, and acetylcholine).

Examples of the UV absorbent include benzophenone-type UV absorbents, such as 2,4-dihydroxybenzophenone, 2,2',4,4'-tetrahydroxybenzophenone, 2-hydroxy-4-methoxybenzophenone, and 2-hydroxy-4-methoxy-4'-methylbenzophenone; benzimidazole-type UV absorbents, such as phenylbenzimidazole-5-sulfonic acid and its salts, phenylenebis-benzimidazole-tetrasulfonic acid and its salts; benzotriazole-type UV absorbents, such as methylenebis-benzotriazolyl-tetramethylbutylphenol; water-soluble UV absorbents, such as 3-benzylidene-d,1-camphor, and urocanic acid.

The amount of component (a) is from 0.1 to 65% by mass relative to the solvent composition, preferably from 15 to 60% by mass, more preferably from 25 to 40% by mass. When the amount is less than 0.1% by mass, then emulsification may be impossible; but on the other hand, when more than 65% by mass, then the emulsion may be unstable to cause phase separation.

The hydrocarbon oil for component (b) includes synthetic and mineral-derived linear or branched, volatile or nonvolatile hydrocarbon oils. Concretely, there may be exemplified volatile liquid paraffins (e.g., isoparaffin and isododecane) or nonvolatile liquid paraffins and their derivatives, liquid paraffins (petrolactams), liquid lanolin, polydecenes, hydrogenated polyisobutene, ceresin, squalene, vaseline, microcrystalline wax, and ozokerite. Above all, preferred are volatile liquid paraffins from the viewpoint of the improvement of the feeling in use (amelioration of gathering and dropping of powder into sulci cutis of the skin, etc.); and more preferred is isododecane.

The silicone oil for component (b) may be any one usable in cosmetics with no specific limitation thereon, for which volatile silicone oils (low-boiling-point silicone oils), nonvolatile silicone oils and the like are widely usable. Also usable are substituent-containing silicone oils and modified silicone oils such as phenyl group-containing silicone oils, fluorine-modified silicone oils, etc. Specific examples of the silicon oil include cyclic silicone oils (cyclomethicones), such as hexamethylcyclotrisiloxane, octamethyltetracyclosiloxane [e.g., "Execol D-4" (by Shin-Etsu Silicone Co., Ltd.), "SH244", "SH344" (both by Dow Corning Toray, Co., Ltd.)], decamethylcyclopentasiloxane [e.g., "Execol D-5" (by Shin-Etsu Silicone Co., Ltd.), "SH245", "DC345" (both by Dow Corning Toray, Co. Ltd.)], dodecamethylcyclohexasiloxane [e.g., "DC246" (by Dow Corning Toray, Co., Ltd.)], and tetradecamethylcycloheptasiloxane; as well as linear silicone oils, such as hexamethyldisiloxane, octamethyltrisiloxane [e.g., "KF-96-1cs" (by Shin-Etsu Chemical Co., Ltd.)], decamethyltetrasiloxane [e.g., "KF-96L-1.5cs" (by Shin-Etsu Chemical Co., Ltd.)], hexadecamethylheptasiloxane, dimethylpolysiloxane [e.g., "KF-96-6cs", "KF-96-10cs", "KF-96-50cs". (all by Shin-Etsu Chemical Co., Ltd.)]. Also usable here are methylphenylpolysiloxanes (liquid) modified from linear silicone oils by substituting a part of the alkyl group therein with a phenyl group.

The amount of component (b) from 35 to 99.9% by mass relative to the solvent composition, preferably from 40 to 85% by mass, more preferably from 58 to 70% by mass. When the amount is less than 35% by mass, then the emulsion may be unstable to cause phase separation; but on the other hand, when more than 99.9% by mass, emulsification may be impossible. In the invention, component (a) is combined with component (b) thereby improving the long lasting performance of the obtained liquid foundation and enhancing the moisturizability thereby.
From the viewpoint of preventing gathering and dropping of powder into sulci cutis of the skin, powdery feeling and others, a film forming agent (c) may be incorporated in the solvent composition of the invention. Component (c) is preferably an oleophilic film forming agent (= oil-soluble polymer) from the viewpoint of the improvement of tensioning feeling, waterproofness improvement, etc. Exemplified preferred ones include silicone-type acrylic polymers (=silicone-modified acrylic polymers), and trimethylsiloxysilicate. Silicone-type acrylic polymers are commercially available as "FA4002ID" (by Dow Corning Toray, Co., Ltd.), etc.; and trimethylsiloxysilicate is commercially available as "X-21-5249" (by Shin-Etsu Chemical), etc. These may be favorably used. Above all, preferred are silicone-type acrylic polymers from the point of feeling in use thereof.

In the case where component (c) is incorporated, its amount is preferably from 0.15 to 15% by mass relative to the solvent composition, more preferably from 0.15 to 7.5% by mass. When the amount is less than 0.15% by mass, component (c) could not exhibit a sufficient effect; but on the other hand, when more than 15% by mass, it tends to occur a sticky feeling.

The solvent composition of the invention may further contain a dispersant as component (d) from the point of storage stability. Not specifically defined, the dispersant may be any one capable of being incorporated in water-in-oil-type emulsion cosmetics, and its preferred examples include esters of mono and/or sesqui-isostearic acid and a polyalcohol having four hydroxyl groups, and silicone-type surfactants.

The ester of mono and/or sesqui-isostearic acid and a polyalcohol having four hydroxyl groups includes sorbitan monoisostearate, sorbitan sesqui-isostearate, diglycerin monoisostearate, and diglycerin sesqui-isostearate. These are commercially available as "Estemol 182V" (by Nisshin Oillio Group, Ltd.), "Emalex SPIS-150" (by Nihon Emulsion Co., Ltd.), etc.; and these are favorably used here.

The silicone-type surfactant includes poly(oxyethylene-oxypropylene)methylpolysiloxane copolymer, polyoxyethylene-methylpolysiloxane copolymer, silicone chain-branched methylpolysiloxane copolymer, alkyl chain-branched polyoxyethylene-methylpolysiloxane copolymer, alkyl chain-silicone chain-branched polyoxyethylene-methylpolysiloxane copolymer, crosslinked polyoxyethylene-methylpolysiloxane, alkyl group-containing crosslinked polyoxyethylene-methylpolysiloxane, branched polyglycerin-modified silicone, crosslinked polyglycerin-modified silicone, alkyl group-containing crosslinked polyglycerin-modified silicone, and alkyl group-branched polyglycerin-modified silicone. Of those, preferred examples include branched polyglycerin-modified silicone, and alkyl group-branched polyglycerin-modified silicone. These are commercially available as laurylpolyglyceryl-3-polydimethylsiloxyethyldimethicone ("KF-6105"), polyglyceryl-3-disiloxanedimethicone ("KF-6100"), polyglyceryl-3-polydimethylsiloxyethyldimethicone ("KF-6104"), polyglyceryl-3-bisbutyldimethicone ("KF-6109") (all by Shin-Etsu Chemical Co., Ltd.), and these are favorably used here.

In the case where a pickering-emulsificated liquid makeup cosmetic (liquid foundation, etc.) is left as such for a long period of time, there may occur a phenomenon of such that the powder gels while precipitated, and even though the cosmetic (container) is stirred by shaking in use thereof, it could not be restored to its original condition (this is referred to as "caking"). By the incorporation of component (d), the occurrence of caking in the obtained converted pickering-emulsificated cosmetic can be more effectively prevented when the cosmetic is left for a long period of time; and even in the case where the composition is mixed just before use, good fit-feeling of the cosmetic to skin is favorably improved.

As component (d), more preferred are esters of mono and/or sesqui-isostearic acid and a polyalcohol having four hydroxyl groups, and polyglyceryl-3-bisbutyldimethicone.

In the case where component (d) is incorporated, its amount is preferably from 0.1 to 10% by mass relative to the solvent composition, more preferably from 0.8 to 10% by mass.

A powdery component may be further incorporated in solvent composition of the invention within a range not detracting from the advantage of the invention, for the purpose of color correction, appearance adjustment, etc. Not specifically defined, the powdery component may be any one capable of being incorporated in ordinary powder makeup cosmetics, and includes body pigments, such as mica, talc, kaolin, calcium carbonate, magnesium carbonate, silicic anhydride, aluminium oxide, and barium sulfate; white pigments, such as titanium dioxide, and zinc oxide; color pigments, such as red iron oxide, yellow iron oxide, black iron oxide, chromium oxide, ultramarine, prussian blue, and carbon black; pearlescent pigments, such as titanium mica, scaly flakes, and bismuth oxychloride. Also usable here are organic powders, such as polyamide resin powder (nylon powder), polyethylene powder, polymethyl methacrylate powder, polystyrene powder, styrene-acrylic acid copolymer resin powder, benzoguanamine resin powder, polytetrafluoroethylene powder, and cellulose powder; organic pigments (e.g., organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, etc.; as well as Red No. 3, Red. No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1, etc.); natural dyes, such as chlorophyll, and β-carotene. The powdery component may be or may be not conducted the surface treatment (e.g., hydrophobication treatment).

Any other component that may be incorporated in ordinary cosmetics may be optionally incorporated in the solvent composition of the invention within a range not detracting from the advantage of the invention. The component that may be incorporated in the composition includes a water-soluble drug (whitening agent, antiinflammatory agent, antimicrobial agent, hormone agent, vitamins, enzyme, antioxidant, plant extracts, etc.), a chelating agent, an aroma chemical, a pH controlling agent, a preservative (phenoxyethanol, etc.). In addition, an oily component (except component (b), such as oils and fats, waxes, higher fatty acids, higher alcohols, and synthetic esters, etc.) and the like may also be incorporated in the composition.

### [Method for conversion into a pickering-emulsificaed liquid cosmetic]

The method for conversion of a powder makeup cosmetic of the invention comprises mixing the above-mentioned solvent composition and a powder makeup cosmetic to thereby convert the powder makeup cosmetic into a pickering-emulsificated liquid makeup cosmetic.

As described above, a purchased, commercial powder makeup cosmetic which has been broken while in use, or of which the remaining part has become small while in use and has remained in the inner peripheral area in the compact case thereof is favorably employed here as the powder makeup cosmetic. An unused powder makeup cosmetic or a powder makeup cosmetic which is in use but will be no more used, for example, for the reason that a foundation having a different color is desired to be used may also be favorably employed here. For converting such a purchased but useless, or unused powder makeup cosmetic into a pickering-emulsificated liquid makeup cosmetic, for example, a cosmetic conversion kit may be used in a simplified manner, which comprises a vessel or container intended for holding a solvent composition therein, and a storage case or container that temporarily stores an unnecessary or unused powder cosmetic, which is to be dispersed in the solvent composition in the vessel ("cosmetic conversion kit of a first embodiment").

One preferred embodiment of the kit for cosmetic conversion is described with reference to the drawing attached hereto. However, the cosmetic conversion kit of the invention is not limited to the illustrated embodiment.

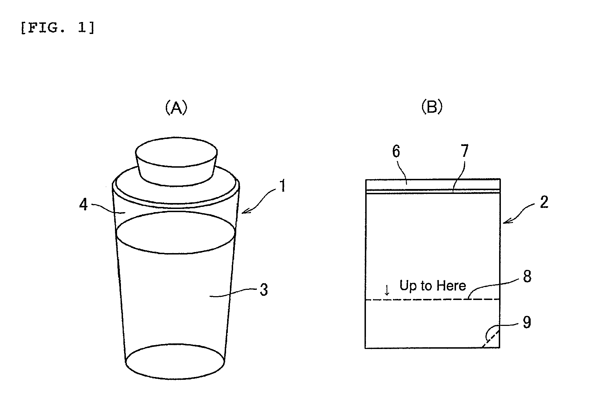

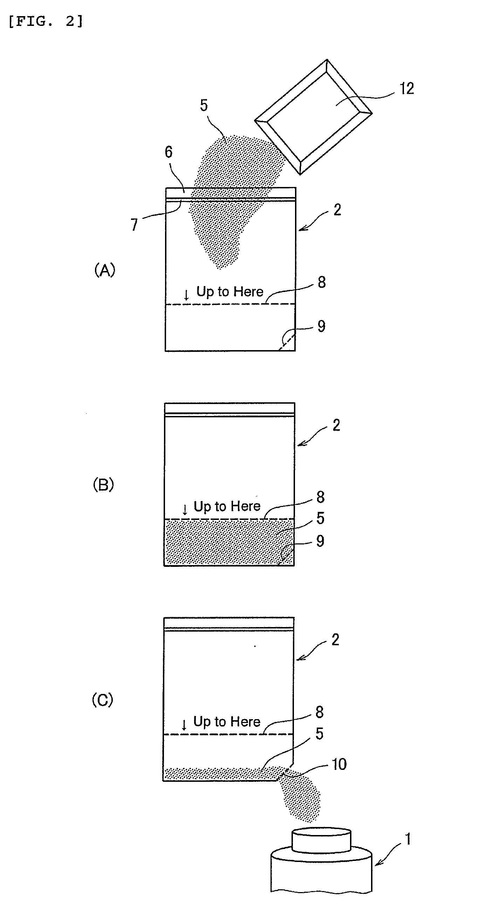

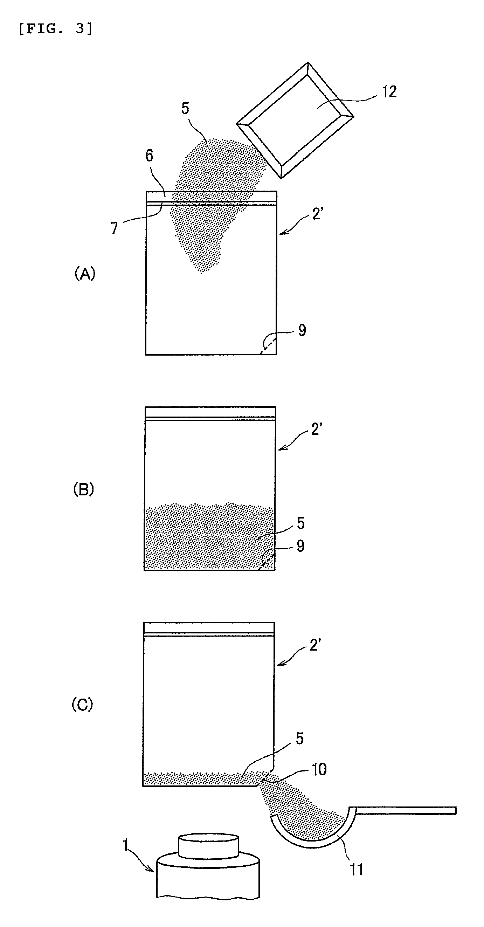

As shown in Fig. 1, the kit for cosmetic conversion for use in the invention comprises a container (bottle) 1 that contains the solvent composition of the invention therein (Fig. 1(A)), and a powder cosmetic storage container 2 (Fig. 1(B)). In the bottle 1, the above-mentioned solvent composition 3 is contained therein beforehand. Preferably, the solvent composition 3 is so put in the bottle 1 as to secure the remaining space 4 enough for stirring and mixing the contents therein when the bottle 1 is shaken after the powder cosmetic 5 (e.g., solidified powder cosmetic such as pressed powder or the like; see Figs. 2 and 3) has been put thereinto. The material of the bottle 1 is not specifically defined, for which preferably used is any material capable of being used as ordinary water-in-oil-type emulsion cosmetic containers (for example, PET, glass, etc.).

The powder cosmetic storage container 2 is one in which the powder cosmetic 5 to be used for cosmetic conversion is temporarily stored therein. The word "temporarily" should not be taken into consideration relating to duration as to either short time or long time. The storage container 2 is not specifically defined, and as illustrated, one preferred example is a rectangular bag having a mouth (opening) 6 at the top thereof. The opening 6 is made openable and closable by the zip fastener (means for opening/closing) 7 or the like. Preferably, the bag-type storage container 2 is given a gauge line 8 or the like that could be a mark to indicate a suitable amount of the powder cosmetic to be added to the solvent composition 3. In the drawing, one example is illustrated with letters of "Up To Here". At one edge of the lower part of the bag, a cutting line 9 is shown by a dotted line or the like so that the bag could be readily cut along the line with scissors or the like or by hand to form the powder discharge port (opening) 10.

An embodiment of a method of using the cosmetic conversion kit is shown in Fig. 2. As illustrated therein, for example, a broken or useless powder cosmetic 5 in a compact case 12 is put into the storage container 2 and temporarily stored therein (Fig. 2(A)). When the powder cosmetic 5 has reached around the gauge line 8, then the cutting line 9 of the container 2 is cut off with scissors or the like, and the powder cosmetic 5 is transferred into the solvent composition 3 in the bottle 1 through the cut-off discharge port (opening) 10, and mixed and stirred therewith to give a pickering-emulsificated liquid cosmetic that has been converted from the powder cosmetic (Fig. 2(B) to (C)).

The storage container 2 may also be a different embodiment not having the gauge line 8. For example, as in Fig. 3, the storage container 2' does not have a gauge line, and instead, it may be combined with a measuring spoon 11. In this case, when the powder cosmetic 5 has been accumulated on some level in the storage container 2, the cutting line 9 of the container 2' is cut off with scissors or the like, and the powder cosmetic 5 is discharged and measured by the measuring spoon 11 through the cut-off discharge port (opening) 10, and thereafter the measured portions is put into the solvent composition 3 in the bottle 1, mixed and stirred therein to give a pickering-emulsificated cosmetic that has been converted from the powder cosmetic (Fig. 3(A) to (C)).

Not giving the cutting line 9 to the container 2, 2', the powder cosmetic may be directly measured and taken out from the container 2, 2' with a measuring spoon 11 or the like through the upper-side mouth 6 by opening the upper-side zip fastener 7 of the container 2, 2', and the thus-measured portion of the powder cosmetic may be added to the solvent composition 3 in the bottle 1, and mixed and stirred therein.

Regarding the amount thereof to be added, the powder cosmetic 5 is preferably so added to the solvent composition that its amount could be from 0.1 to 50% by mass of the entire amount of the pickering-emulsificated liquid cosmetic to be obtained, more preferably from 25 to 45% by mass, even more preferably from 30 to 40% by mass. According to the present invention, the powder cosmetic which is in hand as broken and is useless or which has been purchased but has become unfavorable in point of its color and is now useless with no further consumption can be reused by consumers simply and easily at home, merely by mixing the useless powder cosmetic with the above-mentioned solvent composition, and thus, the useless powder cosmetic can be thereby converted into pickering-emulsificated liquid cosmetic (liquid foundation) excellent in feeling in use, safety and storage stability, and accordingly, the invention contributes toward cutting waste from natural resources and toward protecting the global environment.

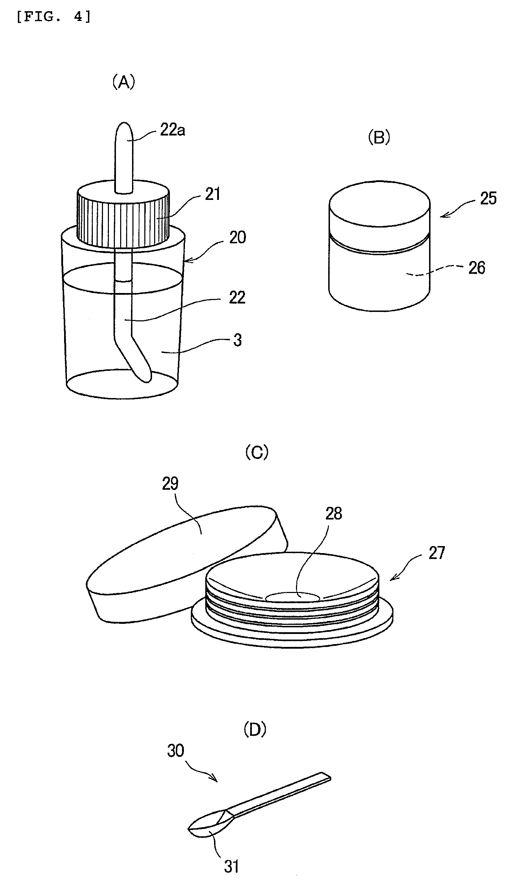

Another embodiment of the cosmetic conversion kit ("cosmetic conversion kit of a second embodiment") is shown in Fig. 4. As illustrated therein, the kit comprises a container (bottle) 20 to contain the solvent composition 3 therein (Fig. 4(A)), a container 25 to contain therein a powder makeup cosmetic (especially, powder-type powdery cosmetic such as loose powder or the like) 26 (Fig. 4(B)), a mixing container 27 to mix the solvent composition 3 and the powder cosmetic 26 each metered in a predetermined amount therein (Fig. 4(C)), and a spatula (mixing member) 30 (Fig. 4(D)). A resin-made dropper member 22 runs through the cap 21 of the container (bottle) 20, and by pushing a few times the upper part 22a of the dropper member 22 with fingers, a necessary amount of the solvent composition 3 can be sucked up and can be thereby metered. The mixing container 27 comprises a container body (saucer) 28 and a lid 29. The spatula (mixing member) 30 is so designed that its tip part 31 has a broadened V-shaped form, and a desired amount of the powder cosmetic 26 can be metered and taken into the V-shaped part 31 from the container 25.

A consumer may buy the kit of the second embodiment, and usually may use the powder cosmetic 26 put in the container 25 that constitutes one part of the kit, as it is therein, but in the winter season, when the consumer desires to use the cosmetic as a liquid foundation or the like, she/he may meter and take the powder cosmetic 26 into the V-shaped part 31 of the spatula 30, and may put it into the saucer 28 of the mixing container 27. On the other hand, a suitable amount of the solvent composition 3 is sucked up with the dropper 22 from the container (bottle) 20, then in that condition, the cap 21 is screwed and opened, and thereafter the solvent composition 3 sucked in the dropper 22 is then dropped down into the saucer 28 and added thereto. With that, in the saucer 28, both the powder cosmetic 26 and the solvent composition 3 are mixed with the spatula 30 to thereby simply and easily prepare a pickering-emulsificated liquid cosmetic (liquid foundation). In this case, the powder cosmetic and the solvent composition may be mixed just before use, or a residual powder cosmetic may be put into the solvent in the bottle and mixed to prepare a pickering-emulsificated liquid cosmetic storable in the bottle. In the case of producing such a storable cosmetic, the blend ratio of the powder cosmetic and the solvent composition is the same as that in the case of the above-mentioned "cosmetic conversion kit of a first embodiment". In the case of mixing the two just before use, the blend ratio of the powder cosmetic to the solvent composition, powder cosmetic/solvent composition is preferably from 2/1 to 6/5 or so. In place of the bottle 20 in Fig. 4, the same one as the bottle 1 in Fig. 1 may also be used.

Regarding the emulsification type thereof, the pickering-emulsificated liquid makeup cosmetic to be obtained according to the invention includes water-in-oil-type (two-layer emulsion type, homogeneous emulsion type or the like) emulsions, and creams etc.

### EXAMPLES

The invention is described in more detail with reference to the following Examples; however, the invention is not limited to the following Examples. The compounding amount is in terms of % by mass.

First described are the evaluation methods employed in the Examples.

### [Storage Stability (caking occurred or not)]

Each test sample was left for 4 weeks (0°C, 50°C), and checked as to whether the precipitated powder gelled and the sample could no more be restored to its original condition when stirred by shaking the container.

### (Evaluation Standards)

Θ: Uniform with no problem.
○: Partly but slightly uneven, but on the level with no problem for practical use.
×: Became uneven.

### [Feeling in use (light spreadability)]

Five woman panelists tried each test sample by applying it onto their cheeks, and checked it for light spreadability thereof.

### (Evaluation Standards)

Θ: Three or more of five panelists answered that the test sample was excellent in light spreadability.
○: Two of five panelists answered that the test sample was excellent in light spreadability.
×: One or less of five panelists answered that the test sample was excellent in light spreadability.

### (Examples 1 to 5)

As in Table 1 below, (1) was added to the solvent composition comprising (2) and (6), mixed and stirred to prepare a liquid foundation (the compounding amount is expressed relative to the entire amount, 100% by mass, of the prepared liquid foundation). The liquid foundation was tested and evaluated for the storage stability (caking occurred or not) and the feeling in use (light spreadability) thereof, according to the methods mentioned above. The results are shown in Table 1.

In Table 1, "silicone-modified acrylic polymer^{(*1)}" is "FA4002ID" (by Dow Corning Toray Co., Ltd.). The same shall apply to Table 2.

**[Table 1]**

| | | Ex.1 | Ex. 2 | Ex. 3 | Ex. 4 | Ex. 5 |
|---|---|---|---|---|---|---|
| (1) Powdery Foundation (commercial product) | | 33.33 | 33.33 | 33.33 | 33.33 | 33.33 |
| (2) Decamethylcyclopentasiloxane | | 45.48 | 44.25 | 43.48 | 45.59 | 39.65 |
| (3) Ion-Exchanged Water | | 16.67 | 16.67 | 16.67 | 16.67 | 16.67 |
| (4) Silicone-Modified Acrylic Polymer^{(*1)} | | 3.75 | 3.75 | 3.75 | 3.75 | 3.75 |
| (5) Sorbitan Sesqui-Isostearate | | 0.77 | - | 0.77 | 0.66 | 6.6 |
| (6) Polyglyceryl-3-Bisbutyldimethicone | | - | 1.5 | 1.5 | - | - |
| Storage Stability (caking occurred or not) | | | | | | |
| | (0°C, 4 weeks) | Θ | ○ | ○ | ○ | ○ |
| | (50°C, 4 weeks) | Θ | ○ | Θ | ○ | ○ |
| Feeling in use (light spreadability) | | Θ | ○ | ○ | ○ | ○ |

As obvious from the results shown in Table 1, the obtained liquid foundations were excellent in the storage stability and the feeling in use (light spreadability). In addition, the liquid foundations were excellent also in the emulsifiability and the applied coating uniformity, and were not observed any gathering and dropping of powder into sulci cutis of the skin.

### (Examples 6 to 7)

As in Table 2 below, (1) was added to the solvent composition comprising (2) and (7), mixed and stirred to prepare a liquid foundation (the compounding amount is expressed relative to the entire amount, 100% by mass, of the prepared liquid foundation). The liquid foundation was tested and evaluated for the storage stability (caking occurred or not) and the feeling in use (light spreadability) thereof, according to the methods mentioned above. The results are shown in Table 2.

**[Table 2]**

| | | Ex. 6 | Ex. 7 |
|---|---|---|---|
| (1) Powdery Foundation (commercial product) | | 33.33 | 33.33 |
| (2) Decamethylcyclopentasiloxane | | 43.78 | 40.48 |
| (3) Ion-Exchanged Water | | 16.67 | 16.67 |
| (4) Silicone-Modified Acrylic Polymer^{(*1)} | | 3.75 | 3.75 |
| (5) Sorbitan Sesqui-Isostearate | | 0.77 | 0.77 |
| (6) Dipropylene Glycol | | 1.5 | 5 |
| (7) Phenoxyethanol | | 0.2 | - |
| Storage Stability (caking occurred or not) | (0°C, 4 weeks) | Θ | Θ |
| | (50°C, 4 weeks) | Θ | Θ |
| Feeling in use (light spreadability) | | Θ | ○ |

As obvious from the results shown in Table 2, even the liquid foundations, in which the preservative ((6), (7)) had been incorporated, were also excellent in the storage stability and the feeling in use (light spreadability). In addition, the liquid foundations were excellent also in the emulsifiability and the applied coating uniformity, and were not observed any gathering and dropping of powder into sulci cutis of the skin.

### (Comparative Examples 1 to 6)

As in Table 3 below, a commercially-available powdery foundation was added to a commercially-available face lotion (water content, 92% by mass; oil content, 8% by mass) or milk (water content, 83% by mass; oil content, 17% by mass) in the ratio indicated below, and mixed and stirred. The resulting mixture was tested and evaluated for the feeling in use (light spreadability) thereof, according to the method mentioned above. The results are shown in Table 3.

**[Table 3]**

| | Comp. Ex.1 | Comp. E. 2 | Comp. Ex. 3 | Comp. Ex. 4 | Comp. Ex. 5 | Comp. Ex. 6 |
|---|---|---|---|---|---|---|
| Powdery Foundation (commercial product) | 50 | 40 | 30 | 50 | 40 | 30 |
| Lotion (commercial product) | 50 | 60 | 70 | - | - | - |
| Emulsion Liquid (commercial product) | - | - | - | 50 | 60 | 70 |
| Feeling in use (light spreadability) | - | × | × | △ | △ | × |

As obvious from the results shown in Table 3, in the case where the commercially-available face lotion or milk was tried for conversion of a powdery foundation to a liquid foundation, a sufficient effect could not be obtained in point of the feeling in use (light spreadability). In Comparative Example 1 the mixture could not be emulsified and therefore could not be tested for the feeling in use.

Compounding Formulation Examples are shown below (the compounding amount is in terms of % by mass).

### (Compounding formulation example 1)

The following powder makeup cosmetic and solvent composition were mixed to give a liquid foundation (final total amount: 100% by mass)

| <Powder makeup cosmetic> | |
|---|---|
| Powdery foundation | 33.33 |

| <solvent composition> | |
|---|---|
| Cyclomethicone | 33.78 |
| Ion-exchanged water | 16.67 |
| Silicone-modified acrylic polymer | 3.75 |
| Sorbitan sesqui-isostearate | 0.77 |
| Dipropylene glycol | 1.5 |
| Phenoxyethanol | 0.2 |
| Trimethylsiloxysilicic acid | 10 |

### (Compounding formulation example 2)

The following powder makeup cosmetic and solvent composition were mixed to give a liquid foundation (final total amount: 100% by mass)

| <Powder makeup cosmetic> | |
|---|---|
| Powdery foundation | 30 |

| <solvent composition> | |
|---|---|
| Cyclomethicone | 44.11 |
| Ion-exchanged water | 16.67 |
| Silicone-modified acrylic polymer | 3.75 |
| Sorbitan sesqui-isostearate | 0.77 |
| Dipropylene glycol | 1.5 |
| Phenoxyethanol | 0.2 |
| Trehalose | 3 |

### (Compounding formulation example 3)

The following powder makeup cosmetic and a solvent composition were mixed to give a liquid foundation (final total amount: 100% by mass)

| <Powder makeup cosmetic> | |
|---|---|
| Powdery foundation | 35 |

| <solvent composition> | |
|---|---|
| Cyclomethicone | 39.11 |
| Ion-exchanged water | 16.67 |
| Silicone-modified acrylic polymer | 3.75 |
| Sorbitan sesqui-isostearate | 0.77 |
| Dipropylene glycol | 1.5 |
| Phenoxyethanol | 0.2 |
| Pearly agent ("SA-flamenco red") | 3 |

### (Compounding formulation example 4)

The following powder makeup cosmetic and a solvent composition were mixed to give a liquid foundation (final total amount: 100% by mass)

| <Powder makeup cosmetic> | |
|---|---|
| Powdery foundation | 40 |

| <solvent composition> | |
|---|---|
| Isododecane | 39.58 |
| Ion-exchanged water | 14.97 |
| Silicone-modified acrylic polymer | 3.75 |
| Dipropylene glycol | 1.5 |
| Phenoxyethanol | 0.2 |

### (Compounding Formulation Example 5)

The following powder makeup cosmetic and a solvent composition were mixed to give a liquid foundation (final total amount: 100% by mass)

| <Powder makeup cosmetic> | |
|---|---|
| Powdery foundation | 43 |

| <Solvent composition> | |
|---|---|
| Octamethyltrisiloxane | 36.58 |
| ("KF-96A-1cs" by Shin-Etsu Chemical Co., Ltd.) | |
| Ion-exchanged water | 14.97 |
| Silicone-modified acrylic polymer | 3.75 |
| Dipropylene glycol | 1.5 |
| Phenoxyethanol | 0.2 |

### (Compounding formulation example 6)

The following powder makeup cosmetic and a solvent composition were mixed to give a liquid foundation (final total amount: 100% by mass)

| <Powder makeup cosmetic> | |
|---|---|
| Powdery foundation | 33.33 |

| <solvent composition> | |
|---|---|
| Dimethylpolysiloxane | 46.25 |
| ("KF-96A-6cs" by Shin-Etsu Chemical Co., Ltd.) | |
| Ion-exchanged water | 14.97 |
| Silicone-modified acrylic polymer | 3.75 |
| Dipropylene glycol | 1.5 |
| Phenoxyethanol | 0.2 |

### INDUSTRIAL APPLICABILITY

The invention makes it possible to simply and easily convert an unnecessary or useless powder makeup cosmetic such as powdery foundation into a pickering-emulsificated liquid makeup cosmetic such as liquid foundation excellent in feeling in use, and therefore contributes toward cutting waste from natural resources and toward protecting the global environment.

### DESCRIPTION OF REFERENCE NUMERALS

- 1, 20: Container (bottle) contained solvent composition
- 2, 2': Powder cosmetic storage container
- 3: Solvent composition
- 4: Space
- 5, 26: Powder Cosmetic
- 6: Mouth
- 7: Zip fastener (means for opening/closing)
- 8: Gauge line
- 9: Cutting line
- 10: Opening
- 11: Measuring spoon
- 12: Compact case
- 21: Cap
- 22: Dropper member
- 25: Container for powder cosmetic
- 27: Mixing container
- 28: Mixing container body (saucer)
- 29: Mixing container lid
- 30: Spatula-like mixing member (spatula)
- 31: Tip of mixing member (V-shaped part)

## Claims

1. A solvent composition for use as a dispersion medium for converting a powder makeup cosmetic into a pickering-emulsificated liquid makeup cosmetic, which comprises:
(a) from 0.1 to 65% by mass of an aqueous component, and
(b) from 35 to 99.9% by mass of one or more oily components selected from hydrocarbon oils and silicone oils.

2. The solvent composition according to claim 1, wherein the hydrocarbon oil in component (b) is a volatile liquid paraffin.

3. The solvent composition according to claim 1, which further comprises (c) a film forming agent.

4. The solvent composition according to claim 3, wherein component (c) is a silicone-type acrylic polymer.

5. The solvent composition as claimed in claim 1 or 3, which further comprises (d) a dispersant.

6. The solvent composition according to claim 5, wherein component (d) is one or more selected from esters of a mono and/or sesqui-isostearic acid and a polyalcohol having 4 hydroxyl groups, and silicone-type surfactants.

7. The solvent composition according to claim 6, wherein the silicone-type surfactant is one or more selected from branched polyglycerin-modified silicone and alkyl group-branched polyglycerin-modified silicone.

8. A method for conversion of a powder makeup cosmetic, which comprises mixing the solvent composition of any one of claims 1, 3 and 5 and a powder makeup cosmetic to thereby convert the powder makeup cosmetic into a pickering-emulsificated liquid makeup cosmetic.

9. A cosmetic conversion kit for converting a powder makeup cosmetic into a pickering-emulsificated liquid makeup cosmetic, which comprises:
a container containing therein the solvent composition of any one of claims 1, 3 and 5, and
a container for a temporarily storage of the powder makeup cosmetic therein.

10. A cosmetic conversion kit for converting a powder makeup cosmetic into a pickering-emulsificated liquid makeup cosmetic, which comprises:
a container containing therein the solvent composition of any one of claims 1, 3 and 5,
a container containing therein a powder makeup cosmetic,
a mixing container for mixing a part or all of the powder makeup cosmetic in the container and the above solvent composition, and
a spatula-like mixing member.
